# NEUE EUROPÄISCHE PATENTSCHRIFT

(11) **EP 3 066 188 B2**
(45) Veröffentlichungstag und Bekanntmachung des Hinweises auf die Entscheidung über den Einspruch: **22.05.2024**
(45) Hinweis auf die Patenterteilung: 11.10.2017
(21) Anmeldenummer: 14802828.5
(22) Anmeldetag: 07.11.2014
(51) Int. Cl.: C12M 1/107, B01F 7/00, B01F 15/00, C12M 1/00, C12M 1/06

(54) **RÜHREINRICHTUNG FÜR EINEN FERMENTER EINER BIOGASANLAGE UND VERFAHREN ZUR HERSTELLUNG EINER RÜHREINRICHTUNG**
AGITATING DEVICE FOR A FERMENTER OF A BIOGAS PLANT AND METHOD FOR PRODUCING AN AGITATING DEVICE
MOYEN D'AGITATION POUR FERMENTEUR D'UNE INSTALLATION DE BIOGAZ ET PROCÉDÉ DE FABRICATION D'UN MOYEN D'AGITATION

(30) Priorität: 08.11.2013 DE 102013018690
(43) Veröffentlichungstag der Anmeldung: 14.09.2016
(73) Patentinhaber: Uts Biogastechnik GmbH, 85399 Hallbergmoos (DE)
(72) Erfinder: CZWALUK, Andreas, 49377 Vechta (DE)
(74) Vertreter: BSB Patentanwälte Schütte & Engelen Partnerschaft mbB
(86) Internationale Anmeldenummer: PCT/EP2014/074025
(87) Internationale Veröffentlichungsnummer: WO 2015/067745

(56) Entgegenhaltungen:
- EP-A1- 0 469 302
- EP-A1- 0 771 586
- EP-A1- 1 876 229
- EP-A2- 0 711 586
- DE-A1- 2 624 991
- KR-B1- 100 970 137
- US-A- 2 794 628
- US-A1- 2006 092 762
- US-A1- 2012 039 721
- US-B1- 6 334 705
- US-B1- 7 473 025

## Beschreibung

Die vorliegende Erfindung betrifft eine Rühreinrichtung für einen Fermenter einer Biogasanlage, einen mit einer solchen Rühreinrichtung ausgerüsteten Fermenter sowie eine solche Biogasanlage und ein Verfahren zur Herstellung einer Rühreinrichtung.

Biogasanlagen weisen regelmäßig einen Fermenter oder mehrere Fermenter auf, in die ein Substrat eingebracht wird, um Biogas zu erzeugen. Dabei ist eine Umrührung des in dem Fermenterinnenraum vorhandenen Substrates erforderlich, um günstige Bedingungen für den Betrieb des Fermenters zu erhalten und zu gewährleisten.

Zum Umrühren werden Rührgeräte bzw. Rühreinrichtungen eingesetzt, die regelmäßig einen oder mehrere Rührflügel aufweisen, um das Substrat entsprechend umzurühren. Die Rührflügel unterliegen einem starken Verschleiß, da die in den Fermentern eingesetzten Substrate sehr abrasiv sein können. Deshalb müssen die Rührflügel regelmäßig ausgetauscht werden. Es sind deshalb Rührgeräte mit Rührflügeln aus Blech bekannt geworden, bei denen das Blech zur Herstellung der Rührflügel abgekantet wird. Dadurch ist die Herstellung einfach und die Kosten für die Rührflügel sind relativ gering.

Mit der KR 100970137 B1 ist ein Rührgerät mit Rührflügeln bekannt geworden, wobei jeder Flügel dreifach abgekantet ist.

Das Rührgerät dient zur Wasserbehandlung und soll Bodensatzablagerungen im Zentrum verhindern. Dazu wird eine Vibration der Flügel im Betrieb reduziert, indem jeweils das radial äußere Ende der Rührflügel parallel zur Achse umgebogen wird.

Die US 2012/0039721 A1 zeigt ein Rührgerät für hydrometallurgische Prozesse mit fünf einfach gestalteten Rührflügeln, die durch ein zweifaches Abkanten der flachen Blechprofile ihre Struktur erhalten. Vorteilhaft daran ist, dass die Herstellkosten relativ gering sind. Für den Einsatz bei hydrometallurgischen Prozessen ist das Rührgerät deshalb geeignet.

Auch die EP 0 771 586 A1 und die DE 26 24 991 A1 betreffen Rührgeräte mit mehreren Rührflügeln.

Bei Biogasanlagen muss der Fermenter aber sehr häufig und meist durchgehend ein oftmals hochviskoses Gemisch umrühren, um die Bildung von Schwimmschichten und das Absetzen bestimmter fester Bestandteile im Fermenter zu verhindern und um Gasblasen nach oben auszutreiben. Ein durchgehender Rührbetrieb im abrasiven Medium ist die Regel. Das Umrühren solcher Medien erfordert sehr viel Energie. Elektrische Leistungen von 16 kW bis 17 kW für ein Rührgerät werden regelmäßig benötigt, wodurch im dauerhaften Rührbetrieb hohe Energiekosten anfallen. Deshalb ist nicht nur der Herstellungsaufwand, sondern auch der Energieaufwand im Betrieb wichtig. Rührgeräte mit abgekanteten Rührflügeln sind zwar günstig in der Herstellung, weisen aber keine günstigen Strömungseigenschaften auf und sind somit teuer im Betrieb, da sie eine geringe Effektivität und einen geringen Wirkungsgrad aufweisen.

Effiziente Rührgeräte weisen propellerartige Rührflügel auf, die dreidimensional strömungsgünstig geformt sind. Derartig geformte und dreidimensional ausgestaltete Rührflügel sind günstiger im Betrieb, da der Energieeinsatz geringer ist, aber solche Rührflügel sind teuer in der Herstellung. Eine Massenproduktion zur Kostensenkung lohnt sich nicht ohne Weiteres, da die Stückzahlen von Rührflügeln zu gering sind.

Es ist deshalb die Aufgabe der vorliegenden Erfindung eine Rühreinrichtung und einen mit einer solchen Rühreinrichtung ausgerüsteten Fermenter sowie ein Verfahren zur Herstellung einer Rühreinrichtung zur Verfügung zu stellen, womit eine wirtschaftlichere Produktion von Biogas ermöglicht wird.

Diese Aufgabe wird gelöst durch eine Rühreinrichtung mit den Merkmalen des Anspruchs 1, sowie durch einen Fermenter mit den Merkmalen des Anspruchs 12 und durch das Verfahren zur Herstellung einer Rühreinrichtung mit den Merkmalen des Anspruchs 13. Weitere Vorteile und Merkmale der vorliegenden Erfindung ergeben sich aus den jeweiligen Unteransprüchen. Außerdem sind weitere Vorteile in der allgemeinen Beschreibung und der Beschreibung des Ausführungsbeispiels angegeben.

Eine erfindungsgemäße Rühreinrichtung ist für den Einsatz in einem Fermenter einer Biogasanlage vorgesehen und umfasst wenigstens einen Rührflügel. Dabei umfasst der Rührflügel eine Vielzahl von zueinander jeweils gewinkelten Flügelabschnitten, um den Rührflügel dreidimensional strömungsgünstig auszubilden. Die Flügelabschnitte sind an den Trennlinien zwischen den Flügelabschnitten umgebogen und so zueinander gewinkelt, dass die Steigung des Rührflügels mit steigendem Abstand von einer zentralen Drehachse abnimmt, um den Rührflügel dreidimensional strömungsgünstig auszubilden, sodass der Vortrieb über der gesamten Querschnittsfläche sich nur wenig ändert. Dabei weisen die Biegekanten in ihrer Ausrichtung neben einer Radialkomponente quer zu einer Drehachse auch eine Axialkomponente parallel zu der Drehachse auf, wobei mit steigendem radialen Abstand einer Biegekante von der Drehachse die Axialkomponente der Biegekante kleiner wird, wobei die Winkelbereiche zwischen zwei benachbarten Biegekanten (37) zwischen 0,5° und 3° liegen. Weiterhin sind wenigstens fünf Flügelabschnitte an dem Rührflügel vorgesehen, wobei sich die Trennlinien auf der Flügelfläche des Rührflügels nicht schneiden.

Die erfindungsgemäße Rühreinrichtung hat viele Vorteile. Ein erheblicher Vorteil der erfindungsgemäßen Rühreinrichtung ist der einfache Aufbau, womit eine einfache Herstellung ermöglicht wird, während gleichzeitig der Betrieb mit einem geringen Energiebedarf ermöglicht wird. Dadurch, dass der Rührflügel eine Vielzahl von zueinander jeweils gewinkelten Flügelabschnitten aufweist, kann eine optimierte und strömungsgünstige Form des Rührflügels erreicht werden. Dabei kann die Form des Rührflügels sowie die Blattneigung und der Durchmesser nach dem Bauraum und der Wunschströmungsrichtung ausgerichtet werden.

Durch die Erfindung wird eine Rühreinrichtung zur Verfügung gestellt, die einfach per Hand oder maschinell unterstützt oder sogar vollautomatisch aus einfachen Komponenten hergestellt werden kann. Dabei wird insbesondere bei Klein- und bei Kleinstserien ein erheblicher Kostenvorteil erzielt, während gleichzeitig eine strömungsgünstige Form ermöglicht wird.

Durch die strömungsgünstige Form kann eine Energieersparnis von 25% und mehr und insbesondere sogar von 33%, 40% und vorzugsweise von 50% und mehr erreicht werden. Es ist möglich, den Leistungsbedarf insgesamt von 16 kW oder 17 kW auf 12 kW und insbesondere auf unter 10 kW zu senken. Durch die Form der Rührflügel wird vermieden, dass einzelne Bereiche der wirksamen Querschnittsfläche gegeneinander arbeiten. Es ist möglich dafür zu sorgen, dass der Vortrieb über der gesamten Querschnittsfläche sich nur wenig ändert. Bei konventionellen Mixern oder Rührern mit sich radial erstreckenden Umbiegekanten wird mit zunehmendem Abstand mehr Vortrieb erzeugt, wodurch es zu erheblichen Reibungsverlusten über der gesamten Querschnittsfläche kommt.

Eine weitere anmeldungsgemäße Rühreinrichtung ist insbesondere für einen Fermenter einer Biogasanlage vorgesehen und weist wenigstens eine um eine Drehachse drehbare Flügelnabe und eine Mehrzahl daran befestigter Rührflügel auf. Jeder Rührflügel umfasst ein Flügelblatt und eine Flügelwurzel insbesondere direkt an der Flügelnabe oder benachbart zu der Flügelnabe. Eine radial innere Flügelfläche und/oder die Flügelfläche an der Flügelwurzel erstreckt sich unter einem Winkel zwischen 15° und 75° zu der Drehachse der Flügelnabe. Dabei besteht das Flügelblatt insgesamt oder doch im Wesentlichen aus einer Vielzahl von zueinander jeweils gewinkelten Flügelabschnitten. Die Flügelabschnitte sind derart zueinander gewinkelt, dass die Steigung des Rührflügels mit steigendem Abstand von einer zentralen Drehachse abnimmt, um den Rührflügel dreidimensional strömungsgünstig auszubilden.

In allen Weiterbildungen und Ausgestaltungen sind die Flügelabschnitte eines Rührflügels vorzugsweise insgesamt einstückig miteinander verbunden.

Es ist besonders bevorzugt, dass die Flügelabschnitte eines Rührflügels durch vielfaches Umbiegen eines flachen Grundkörpers gebildet sind. Der Grundkörper kann dabei beispielsweise aus einem metallischen Material und insbesondere einem Blechmaterial bestehen. Beispielsweise ist es möglich die Flügelabschnitte eines Rührflügels und den Rührflügel praktisch insgesamt aus einer ursprünglich flachen Stahlplatte zu formen, die an den Rändern der jeweiligen Flügelabschnitte umgebogen oder abgekantet wird.

Es ist besonders bevorzugt, dass die Flügelabschnitte jeweils im Wesentlichen eben ausgebildet sind. Das bedeutet, dass insbesondere im Wesentlichen jeder Flügelabschnitt und vorzugsweise jeder Flügelabschnitt eine flache Struktur aufweist.

Insbesondere weist wenigstens ein Teil der Vielzahl der Flügelabschnitte jeweils eine im Wesentlichen flache Struktur auf. Insbesondere weisen im Wesentlichen alle Flügelabschnitte und insbesondere alle Flügelabschnitte eine flache Struktur auf. Unter einer flachen Struktur wird im Sinne der vorliegenden Erfindung eine Struktur verstanden, die auf ihrer Fläche nur unwesentlich ihre Höhe ändert. Vorzugsweise ist die Höhe einer flachen Struktur kleiner als die vierfache Materialstärke. Die Höhe der Struktur kann auch kleiner sein als die zweifache Materialstärke.

Insbesondere ergibt sich für wenigstens einen Teil der Flügelabschnitte eine im Wesentlichen viereckige Struktur. Die Flügelabschnitte sind dabei insbesondere streifenförmig ausgebildet. Möglich sind auch dreieckige Flügelabschnitte und auch rechteckige Flügelabschnitte. Rechteckige Flügelabschnitte werden eher seltener eingesetzt, da durch Flügelabschnitte, die an einem Ende spitz zusammenlaufen, insgesamt eine geeignete dreidimensionale Struktur erzielt werden kann.

In bevorzugten Ausgestaltungen weist wenigstens einer der Flügelabschnitte eine Höhe quer zu seiner Flügelfläche auf, die kleiner als eine vierfache Materialstärke des Flügelabschnitts ist. Diese Angabe gilt insbesondere jeweils auch für die Vielzahl der Flügelabschnitte.

Die Rührflügel sind dreidimensional ausgeformt. Die Rührflügel erstrecken sich auch in die axiale Richtung der Drehachse und auch in radialer Richtung. Ein an der Flügelnabe montierter Rührflügel erstreckt sich vorzugsweise in axialer Richtung (axiale Erstreckung) über wenigstens 1/6 einer maximalen radialen Erstreckung des Rührflügels von der Drehachse aus. Insbesondere beträgt die axiale Erstreckung wenigstens 1/5 einer radialen Erstreckung des Flügelblatts.

In allen Ausgestaltungen ist es bevorzugt, dass jeweils zwei benachbarten Flügelabschnitten jeweils eine dazwischen angeordnete und im Wesentlichen linear verlaufende Biegekante definiert wird und/oder angeordnet ist.

Erfindungsgemäß weisen die Biegekanten in ihrer Ausrichtung neben einer Radialkomponente quer zu einer Drehachse auch eine Axialkomponente parallel zu der Drehachse der Flügelnabe auf, wobei mit steigendem radialen Abstand einer Biegekante von der Drehachse, die Axialkomponente der Biegekante kleiner wird.

Erfindungsgemäß sind wenigstens fünf Flügelabschnitte an dem Rührflügel vorgesehen sind. Möglich ist es auch, dass 10, 12, 14 oder noch mehr Flügelabschnitte vorgesehen sind. Mit der Anzahl der Flügelabschnitte steigt auch die Anpassungsmöglichkeit der dreidimensionalen Struktur, wobei eine höhere Anzahl an Flügelabschnitten eine bessere Übereinstimmung mit der theoretisch idealen Geometrie des Rührflügels erlaubt. Andererseits steigt mit der Anzahl der Flügelabschnitte auch der Herstellungsaufwand, sodass sich eine Zahl zwischen 5 und 20 Flügelabschnitten und insbesondere zwischen 6 und 15 Flügelabschnitten als positiv herausgestellt hat. Die Anzahl und Art der Flügelabschnitte hängt auch vom vorgesehenen Einsatzzweck und von der Materialstärke und der Materialart des Rührflügels ab.

Vorzugsweise ist wenigstens ein Winkel zwischen zwei benachbarten Biegekanten größer als 1°. Vorzugsweise sind alle Winkel zwischen jeweils zwei benachbarten Biegekanten größer als 0,5° und insbesondere größer als 1°. Benachbarte Biegekanten verlaufen vorzugsweise fächerartig auseinander.

In allen Ausgestaltungen ist es möglich und bevorzugt, dass wenigstens ein Flügelabschnitt abgekantet ist.

Die Steigung des Rührflügels nimmt mit steigendem Abstand von einer zentralen Drehachse der Rühreinrichtung ab. Bei Rühreinrichtungen für Fermenter für Biogasanlagen ist es vorteilhaft, wenn der Vortrieb über den gesamten Durchmesser der Rühreinrichtung bzw. des Propellers in etwa gleich ist. Dadurch ergibt sich, dass der lokale Winkel der Rührflügel bzw. der Winkel der Blattstellung zu der zentralen Drehachse von dem jeweiligen Durchmesser abhängt. Das bedeutet, dass der Winkel des Rührflügels zu der zentralen Achse bzw. der Rotationsachse nahe am Zentrum steiler ist als radial weiter außen. Deshalb ist es bevorzugt, dass ein Produkt eines Abstandes von einer zentralen Drehachse und der lokalen Steigung des Rührflügels sich über der Flügelfläche um weniger als den Wert 10 und insbesondere um weniger als den Wert 4 und vorzugsweise weniger als den Wert 2 oder sogar 1,5 ändert. Dadurch wird für einen relativ gleichen Vortrieb über dem gesamten Durchmesser der Rühreinrichtung gesorgt.

Vorzugsweise ist ein lokaler Neigungswinkel an einer Oberflächenstelle des Rührflügels relativ zu einer Achse, die durch die Oberflächenstelle verläuft und die quer zu der zentralen Drehachse ausgerichtet ist abhängig von dem Abstand von der zentralen Drehachse. Dabei nimmt der lokale Neigungswinkel vorzugsweise mit zunehmendem Abstand von der zentralen Drehachse ab.

In bevorzugten Weiterbildungen ist der Rührflügel an einer Aufnahmeeinrichtung befestigt. Die Aufnahmeeinrichtung kann grundsätzlich beliebig gestaltet sein und ist in einer konkreten Ausführungsform als gewinkeltes Aufnahmeblech ausgeführt. Dadurch wird ebenfalls eine einfachere Fertigung erreicht.

In allen Ausgestaltungen ist es besonders bevorzugt, dass eine Mehrzahl von wenigstens zwei oder drei oder mehr Rührflügeln vorgesehen ist. Diese sind insbesondere symmetrisch über dem Umfang angeordnet.

Besonders bevorzugt ist es, dass die Aufnahmeeinrichtungen der Rührflügel insgesamt eine mehrkantige Achsenaufnahme bilden. Beispielsweise können die Aufnahmeeinrichtungen von drei Rührflügeln insgesamt eine sechskantige Außenoberfläche bilden, wobei jeder Rührflügel an zwei Außenkanten über ein gewinkeltes Aufnahmeblech befestigt ist.

Besonders bevorzugt ist der Rührflügel über wenigstens ein lösbares Verbindungsmittel an der Aufnahmeeinrichtung oder an einer separaten Achsenaufnahme befestigt. Ein solches lösbares Verbindungsmittel kann beispielsweise eine Schraube sein.

In bevorzugten Ausgestaltungen umfasst die Rühreinrichtung eine Antriebseinrichtung und wenigstens eine Antriebswelle. Der Rührflügel kann dabei mit einer Antriebswelle wenigstens im Wesentlichen drehfest verbunden sein. Aus Fertigungsgründen oder auch aus anderen Gründen kann es möglich sein, dass der Rührflügel ein gewisses Drehspiel aufweist. Die Antriebseinrichtung kann grundsätzlich beliebig ausgeführt sein. Möglich und bevorzugt sind elektrische Antriebseinrichtungen und auch hydraulische Antriebseinrichtungen.

Ein erfindungsgemäßer Fermenter für eine Biogasanlage umfasst wenigsten eine Fermenterwandung und wenigstens einen Fermenterinnenraum. Wenigstens ein Fermenterdach ist vorgesehen. Die Fermenterwandung und das Fermenterdach können aus unterschiedlichsten Materialien bestehen. Möglich sind Fermenter aus Beton oder ähnlichen Materialien. Möglich sind auch Fermenter aus Stahl. Das Fermenterdach kann als massives Dach ausgebildet sein oder auch als flexibles Foliendach vorgesehen sein. In dem Fermenterinnenraum ist wenigstens eine Rühreinrichtung angeordnet, wobei die Rühreinrichtung wenigstens einen Rührflügel umfasst. Dabei umfasst der Rührflügel eine Vielzahl von zueinander jeweils gewinkelten Flügelabschnitten, um den Rührflügel dreidimensional strömungsgünstig auszubilden. Die Rühreinrichtung des erfindungsgemäßen Fermenters ist ausgebildet, wie eine zuvor beschriebene erfindungsgemäße Rühreinrichtung oder eine der Weiterbildungen.

Auch der erfindungsgemäße Fermenter hat viele Vorteile. Ein solcher Fermenter ermöglicht einen zuverlässigen und wenig Energie benötigenden Betrieb, wobei die Rührflügel als Verschleißteile kostengünstig nachbeschafft werden können. Weiterhin sind die Rührflügel vorzugsweise separat befestigt und insofern einfach sogar einzeln austauschbar.

Ein erfindungsgemäßes Verfahren dient zur Herstellung einer Rühreinrichtung insbesondere für einen Fermenter einer Biogasanlage, wie sie zuvor beschrieben wurde, wobei die Rühreinrichtung wenigstens eine Mehrzahl von Rührflügeln umfasst. Dabei wird ein Rührflügel aus einer Vielzahl von jeweils zueinander gewinkelten Flügelabschnitten gebildet. Die Flügelabschnitte der Rührflügel werden an den Trennlinien zwischen den Flügelabschnitten so zueinander gewinkelt umgebogen, dass die Steigung des Rührflügels mit steigendem Abstand von einer zentralen Drehachse abnimmt, um den Rührflügel dreidimensional strömungsgünstig auszubilden. Dabei weisen die Biegekanten in ihrer Ausrichtung neben einer Radialkomponente quer zu einer Drehachse auch eine Axialkomponente parallel zu der Drehachse auf, wobei mit steigendem radialen Abstand einer Biegekante von der Drehachse die Axialkomponente der Biegekante kleiner wird. Weiterhin ist wenigstens ein Winkel zwischen zwei benachbarten Biegekanten größer als 1°.

Das erfindungsgemäße Verfahren hat viele Vorteile, da es die einfache und kostengünstigere Herstellung eines Rührflügels erlaubt, der weiterhin einen energetisch günstigen Betrieb ermöglicht. Ein solcher Rührflügel kann auch in Kleinst-und Kleinserien kostengünstig gefertigt werden. Je nach Größe der Serie bietet sich eine Handfertigung oder eine teil- oder vollautomatisierte Fertigung an.

Vorzugweise wird der Rührflügel aus einem flächigen Grundkörper durch Umbiegen an den einzelnen Flügelabschnitten erzeugt. Dabei wird der zunächst flächige Grundkörper, wie beispielsweise eine flachliegende Stahlplatte, zunächst in der gewünschten Abwicklung ausgeschnitten und anschließend werden an dem Grundkörper die einzelnen Flügelabschnitte durch Umbiegen erzeugt.

Der vorzugsweise flächige Grundkörper wird an einer Trennlinie zwischen den Flügelabschnitten umgebogen und insbesondere abgekantet. Durch mehrmaliges nacheinander erfolgendes Umbiegen bzw. Abkanten der jeweiligen Flügelabschnitte kann die gewünschte dreidimensionale Form stark angenähert werden. Das Produkt erfüllt die gewünschten Anforderungen an den Volumenstrom, die Strömungsrichtung und den Energiebedarf.

In allen Ausgestaltungen ist es bevorzugt, dass über lösbare Verbindungsmittel wenigstens mehrere Rührflügel mittelbar oder unmittelbar miteinander verbunden werden. Dabei können die Rührflügel an Aufnahmeeinrichtungen in Form von beispielsweise gewinkelten Aufnahmeblechen angebracht werden, wobei die Aufnahmeeinrichtungen wiederum direkt oder indirekt miteinander verbunden werden.

Die Struktur des Rührflügels ist dabei derart, dass ein Winkel zwischen zwei Flügelabschnitten von einem Abstand von einer Drehachse abhängt.

In allen Ausgestaltungen erfolgt die Auswahl des Durchmessers des einen Rührflügels oder der Vielzahl der Rührflügel in Abstimmung mit dem Bauraum. Die Auswahl der Blattneigung des Rührflügels erfolgt nach der gewünschten Strömungsrichtung. Die Auswahl der Steigung kann nach dem Verhältnis zwischen Schub- und eingesetzter Energie oder auch nach dem Volumenstrom im Verhältnis zur eingesetzten Energie erfolgen. Die Steigung, d. h. der Vortrieb, wird vorzugsweise derart gewählt, dass der Vortrieb über den ganzen Durchmesser der Rühreinrichtung in etwa gleich ist. Dadurch ergibt sich ein Winkel der Blattstellung des Rührflügels zur rotierenden Achse, der je nach lokalem Durchmesser unterschiedlich ist, wobei der Winkel steiler an der Nabe und flacher am Außendurchmesser ist.

Die Abwälzungskurve zur Anfertigung des Grundkörpers wird in Drehrichtung so gewählt, dass die Abwälzung verzopfungsfrei funktioniert. Als Ergebnis ergibt sich eine Freiformfläche in 3D, die über Biegeverfahren gut angenähert werden kann.

Der Blechzuschnitt für den Grundkörper und andere Teile erfolgt so, dass ein vorrichtungsarmes Fügen vorgesehen wird. Dabei können Konturen und Gravuren aufgebracht werden, um die Kantlinien, die vorgesehenen Schritte und die umzubiegenden Winkel auf dem Grundkörper aufzutragen, sodass die nachfolgenden Fertigungsschritte eindeutig erkennbar sind.

Zur Fertigstellung ist eine Vorrichtung möglich, an der die Lage der einzelnen Bauteile eindeutig positionierbar ist, um so auch eine Sichtprüfung der Geometrie nachvollziehen zu können.

Weitere Vorteile und Anwendungsmöglichkeiten der vorliegenden Erfindung ergeben sich aus dem Ausführungsbeispiel, welches im Folgenden mit Bezug auf die beiliegenden Figuren erläutert wird.

In den Figuren zeigen:
- Fig. 1: eine schematische Seitenansicht eines erfindungsgemäßen Fermenters;
- Fig. 2: einen schematischen Querschnitt durch das obere Ende der Fermenterwand und eine relativ weit nach oben gezogene erfindungsgemäße Rühreinrichtung;
- Fig. 3: eine perspektivische Darstellung einer erfindungsgemäßen Rühreinrichtung;
- Fig. 4: eine perspektivische explodierte Darstellung einer erfindungsgemäßen Rühreinrichtung;
- Fig. 5: den Grundkörper eines Rührflügels der erfindungsgemäßen Rühreinrichtung in der Draufsicht und der Seitenansicht; und
- Fig. 6: den aus dem Grundkörper nach Fig. 5 erzeugten Rührflügel.

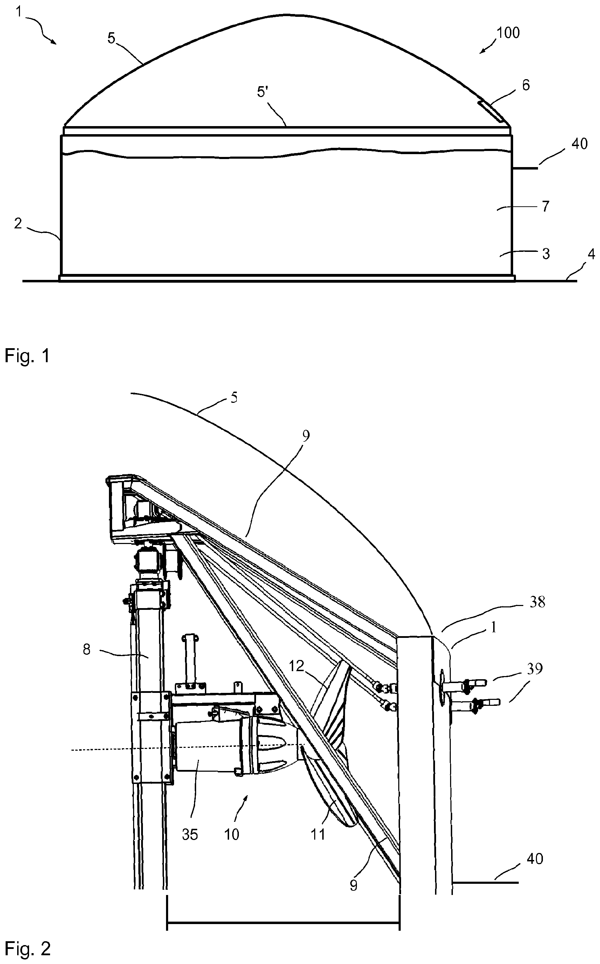

Mit Bezug auf die Figuren wird nun ein Ausführungsbeispiel erläutert. Dabei zeigt Figur 1 in einer stark schematischen Seitenansicht einen Fermenter 1 einer Biogasanlage 100. Der Fermenter 1 weist vorzugsweise einen etwa kreisförmigen Querschnitt auf und verfügt über eine hier ringsum umlaufende Fermenterwandung 2 aus z. B. Beton oder Stahl. Das Fermenterdach 5 kann ebenso wie der Boden als flaches Stahl- oder Betondach 5' ausgeführt sein. Hier wird das Fermenterdach 5 durch ein insbesondere flexibles Material gebildet und erstreckt sich von der Wandung aus nach oben, sodass sich eine gewölbte Struktur des Behälterdaches 5 ergibt. Der Neigungswinkel des Fermenterdaches 5 hängt von den konkreten Bedingungen ab und kann 15° oder mehr und insbesondere auch 30 oder 45° erreichen oder überschreiten. Vorzugsweise ist das Fermenterdach 5 wenigstens teilweise und insbesondere vollständig entfernbar, um den Fermenterinnenraum 3 zugänglich zu machen.

In dem Fermenterinnenraum 3 ist im Betrieb ein Substrat 7 vorgesehen.

In dem Fermenterdach 5 kann wenigstens eine Serviceöffnung 6 vorgesehen sein, um beispielsweise ein in dem Fermenterinnenraum 3 angeordnetes Rührgerät 10 zu warten. Eine Plattform kann beispielsweise an der Außenseite der Fermenterwandung 2 befestigt sein, damit eine Bedienperson sich darauf stellen kann.

Figur 2 zeigt einen Teilquerschnitt durch die Fermenterwandung 2, wobei die Rühreinrichtung 10 mit zwei der daran vorgesehenen Rührflügeln 11-13 sichtbar ist.

Die Rühreinrichtung 10 ist als Tauchmotorrührgerät ausgeführt und kann elektrisch oder beispielsweise auch hydraulisch angetrieben werden. Das schematisch dargestellte Rührgerät 10 verfügt hier im Ausführungsbeispiel über eine Mehrzahl von z. B. drei Rührflügeln 11-13, die sich im Betrieb der Rühreinrichtung drehen und somit für eine Durchmischung des Substrates 7 in dem Fermenterinnenraum 3 führen. Dadurch werden eine Homogenisierung und eine bessere Effektivität ermöglicht.

Das Rührgerät 10 ist an einer als Stützmast oder Hohlprofil ausgebildeten Führungseinheit 8 höhenverstellbar gehalten.

Das Rührgerät 10 kann aus der in Figur 2 dargestellten Position soweit nach oben gefahren werden, bis es an dem oberen Ende der Führungseinheit 8 anschlägt. Die Rühreinrichtung 10 kann nach unten entlang der Führungseinheit 8 abgesenkt werden, bis die Rührflügel 11 bis 13 nur noch einen kurzen Abstand oberhalb des Fermenterbodens erreichen.

Das Rührgerät 10 ist hier zusammen mit dem etwa rechteckig ausgebildeten Stützmast bzw. der etwa rechteckig oder mit einem sonstigen Querschnitt ausgebildeten Führungseinheit 8 verschwenkbar vorgesehen. Zur Verschwenkung des Rührgeräts 10 und zur Einstellung eines Rührwinkels kann die Führungseinheit 8 um ihre Längsachse gedreht werden. Dadurch wird es ermöglicht, dass das Rührgerät 10 in jede beliebige Richtung ausrichtbar ist.

Die Führungseinheit 8 ist hier über Stützeinheiten 9 an der Innenseite der Fermenterwandung 2 befestigt und gehalten. Dabei umfassen die Stützeinheiten 9 jeweils eine oder mehrere Haltestangen, die hier jeweils schräg nach oben verlaufen. Dadurch wird eine effektive Abstützung der Führungseinheit 8 ermöglicht.

Dadurch, dass in der Wartungsstellung die Rührflügel 11 bis 13 wenigstens teilweise über das obere Ende 38 der Fermenterwandung 2 hinausragen, kann ein auf der Plattform 40 stehender Servicetechniker die Rührflügel 11 bis 13 relativ bequem und sogar einzeln tauschen.

Um eine Höhe des Rührgeräts 10 einzustellen, und um einen Schwenkwinkel des Rührgeräts 10 einzustellen, sind Einstelleinheiten 39 vorgesehen.

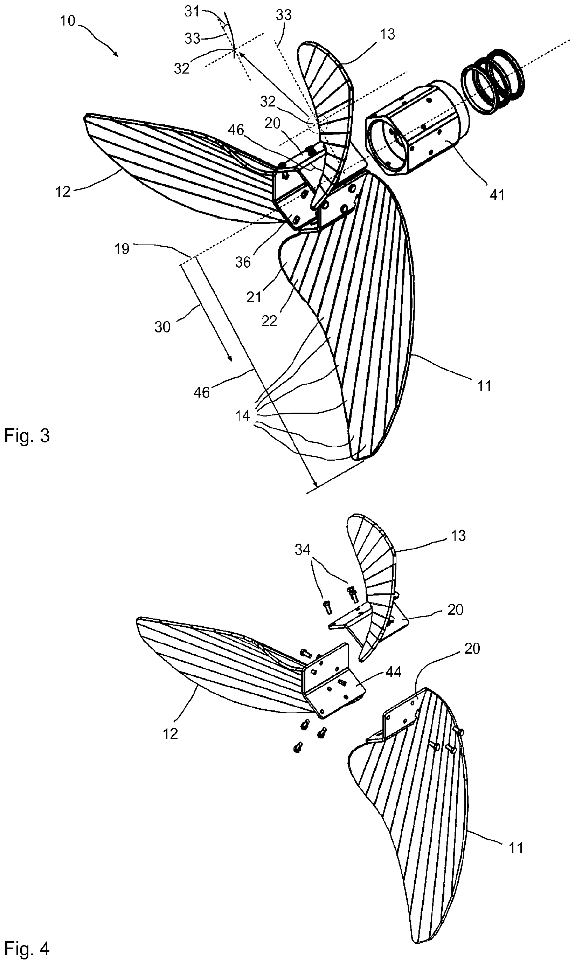

Wie in Figur 2 schematisch zu erkennen, weisen die Rührflügel 11, 12 jeweils eine Vielzahl von Flügelabschnitten auf. Die Struktur der Rührflügel 11 bis 13 und deren Aufbau wird im Folgenden mit Bezug auf die Figuren 3 und 4 näher beschrieben.

Figur 3 zeigt dabei eine perspektivische Darstellung der Rühreinrichtung 10, die hier mit drei Rührflügeln 11, 12 und 13 ausgerüstet ist.

Jeder Rührflügel 11 bis 13 ist hier an einer Aufnahmeeinrichtung 20 befestigt. Die Aufnahmeeinrichtung 20 der drei Rührflügel 11 bis 13 stellt insgesamt eine Achsenaufnahme 36 zur Verfügung, die zur Aufnahme der Antriebswelle des Rührgeräts dient. Die Antriebswelle wird dabei in der Nabe 41 befestigt, die in die Achsenaufnahme 36 eingeschoben und damit verschraubt wird. Damit sind die drei Rührflügel 11, 12, 13 einzeln austauschbar. Zum Austausch werden die Schrauben als Verbindungsmittel 34 des entsprechenden Rührflügels 11 bis 13 gelöst, sodass der jeweilige Rührflügel entfernt und ausgetauscht werden kann.

Jeder Rührflügel 11 bis 13 umfasst eine Vielzahl 14 von Flügelabschnitten 21, 22ff., die hier jeweils aus Ebenen und flachen Segmenten bestehen.

Die Rühreinrichtung 10 ist um die zentrale Drehachse 19 drehbar. Mit dem Abstand 30 von der zentralen Drehachse 19 nimmt die Steigung des Rührflügels ab. Dadurch wird ein im Wesentlichen gleicher Vortrieb über der Querschnittsfläche der Rühreinrichtung 10 ermöglicht.

Dazu ist an einer Oberflächenstelle 32 der Neigungswinkel 31 gegenüber einer durch die Oberflächenstelle 32 laufenden Achse 33, die quer zur zentralen Drehachse 19 ausgerichtet ist, abhängig von dem Abstand 30 zur zentralen Drehachse. Dabei gilt: je größer der Abstand 30 desto kleiner der Neigungswinkel 31.

Die Rührflügel der Rühreinrichtung 10 sind an einer um eine Drehachse 19 drehbaren Flügelnabe 41 befestigt. Die bzw. jeder jeder Rührflügel 11-13 umfasst ein Flügelblatt 43 und eine Flügelwurzel 44 benachbart zu der Flügelnabe 41. Hier reichen die Flügelblätter 43 direkt bis zur Flügelnabe 41. Die radial innere Flügelfläche 45 erstreckt sich unter einem Winkel 46 zwischen 15° und 75° und insbesondere zwischen etwa 25° und 60° zu der Drehachse 19 der Flügelnabe 41.

Wenigstens einer der Rührflügel und insbesondere alle an der Flügelnabe 41 montierte Rührflügel 11-13 erstrecken sich in die axiale Richtung der Drehachse 19 über wenigstens 1/6 und insbesondere mehr als 1/5 einer maximalen radialen Erstreckung 46 des Rührflügels von der Drehachse 19 aus.

Das bedeutet, dass die Rührflügel an der Flügelwurzel schräg zu der Flügelnabe 41 verlaufen. Die Flügelabschnitte des Rührflügels 11-13 sind derart zueinander gewinkelt, dass die Steigung des Rührflügels 11-13 mit steigendem Abstand 30 von einer zentralen Drehachse 19 abnimmt, um die Rührflügel 11-13 jeweils dreidimensional strömungsgünstig auszubilden.

Figur 4 zeigt eine explodierte perspektivische Ansicht der Rühreinrichtung 10 mit den drei Rührflügeln 11, 12 und 13. Die einzelnen Rührflügel 11 bis 13 sind an den jeweiligen Aufnahmeeinrichtungen 20 hier im Ausführungsbeispiel angeschweißt. Möglich und denkbar sind auch andere Befestigungsmöglichkeiten. Die Aufnahmeeinrichtungen 20 sind wiederum über die hier als Schrauben ausgeführten Verbindungsmittel 34 mit der Nabe 41 verschraubt.

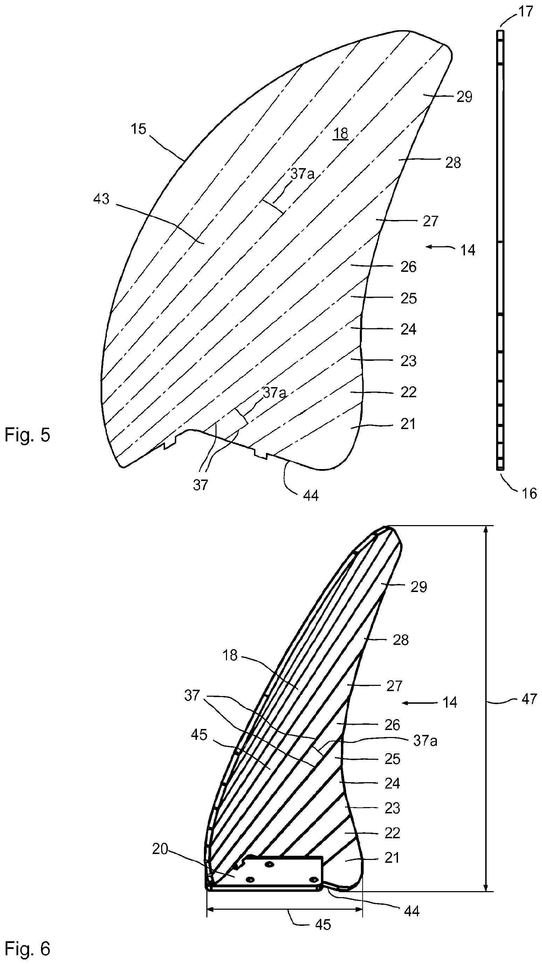

Figur 5 zeigt den Grundkörper 15 für einen Rührflügel 11 bis 13, bevor der Rührflügel seine endgültige Gestalt erhalten hat. Die Rührflügel 11 bis 13 werden hier jeweils aus einer flachen Stahlplatte oder dergleichen hergestellt und weisen hier zunächst eine flache und ebene Struktur mit konstanter Wandstärke über der Fläche auf. Die Abwicklung des Rührflügels 11 bis 13 wird aus einer flachliegenden Stahlplatte ausgeschnitten, sodass sich ein Grundkörper 15 ergibt, wie er in Figur 5 in der linken Hälfte in der Draufsicht dargestellt ist.

Beispielsweise ist es möglich, den Grundkörper 15 durch Laserschneiden auszuschneiden. Mit dem Laser oder über andere Hilfsmittel können auf der Flügelfläche 18 des Rührflügels zusätzliche Markierungen und Angaben aufgebracht werden. Beispielsweise werden die einzelnen Flügelabschnitte 21 bis 29ff. auf dem Grundkörper 15 vorgezeichnet. Dadurch werden schon beim Ausschneiden des Grundkörpers 15 entsprechende Biegelinien auf der Flügelfläche aufgezeichnet. Weiterhin können Angaben zu den jeweiligen Biegewinkeln und über die Reihenfolge der Verfahrensschritte dort schriftlich hinterlegt werden, sodass bei der Bearbeitung per Hand die Bedienperson jederzeit über die Art und Reihenfolge der nächsten Arbeitsschritte informiert ist.

Die Anzahl der Flügelabschnitte 21ff. hängt vom Einsatzzweck und den Anforderungen ab. Hier weist der dargestellte Rührflügel insgesamt etwa 13 unterschiedliche Flügelabschnitte auf, von denen nur die Flügelabschnitte 21 bis 29 mit Bezugszeichen versehen sind. Die jeweiligen Flügelabschnitte sind streifenartig ausgebildet, wobei die Trennlinien 37 zwischen den einzelnen Flügelabschnitten linear verlaufen, aber jeweils zueinander Winkel 37a haben können. Dadurch wird ein einfaches Biegeverfahren zur Herstellung des fertigen Rührflügels ermöglicht. Die jeweilige Trennlinie 37 muss passend an der Biegemaschine oder der Abkantmaschine angelegt oder angeordnet werden, bevor der passende Winkel umgebogen wird. Nach der Durchführung der Vielzahl an Biegevorgängen wird die in Figur 6 abgebildete Struktur erhalten, bei der sich der Rührflügel nach vorn aus der Blattebene heraus erstreckt. Jeder einzelne Flügelabschnitt 21ff. weist dann eine im Wesentlichen ebene Flügelfläche 18 auf. Eine dreidimensionale und strömungsgünstige Struktur entsteht durch die Vielzahl der jeweils umgebogenen Flügelabschnitte. Dadurch wird eine ideale Rührflügelkontur stark angenähert. Gleichzeitig ermöglicht diese Fertigungsart eine günstige Produktion der Rührflügel, die aufgrund des begrenzten Anwendungsgebietes nur in Klein- oder Kleinstserien in der Regel wenigstens teilweise manuell hergestellt werden.

Zwischen einzelnen Flügelabschnitten 21-29 verlaufen Trennlinien oder Biegekanten 37, an denen die einzelnen Flügelabschnitte 21-29 umgebogen und/oder abgekantet werden. Die Trennlinien 37 sind bei den fertigen Rührflügeln Biegekanten 37, die im Wesentlichen oder vollständig linear zwischen zwei benachbarten Flügelabschnitten verlaufen.

Die Biegekanten 37 weisen in ihrer Ausrichtung neben einer Radialkomponente quer zu der Drehachse 19 auch eine Axialkomponente parallel zu der Drehachse 19 auf. Mit steigendem radialen Abstand einer Biegekante 37 von der Drehachse 19 wird die Axialkomponente des Verlaufs der Biegekante 37 kleiner.

Ein Winkel zwischen zwei benachbarten Biegekanten 37 z. B. der Flügelabschnitte 26, 27 und 27, 28 beträgt vorzugsweise größer als 1°. Erfindungsgemäß sind alle Winkel zwischen jeweils zwei benachbarten Biegekanten größer als 0,5° und insbesondere größer als 1°. Erfindungsgemäße Winkelbereiche liegen zwischen 0,5° und 3°. Benachbarte Biegekanten verlaufen, wie in Fig. 5 dargestellt, vorzugsweise fächerartig auseinander.

Wie dem rechten Teil der Figur 5 entnehmbar ist, die eine Seitenansicht des Grundkörpers 15 zeigt, weist der Grundkörper eine Materialstärke 16 mit einer Höhe 17 auf, die praktisch in jedem Flügelabschnitt 21ff. erhalten bleibt. Nur an den jeweiligen Rändern und Biegekanten der Flügelabschnitte kann durch die Biegungen bedingt eine geringfügig größere Höhe entstehen. Die Höhe 17 eines Flügelabschnitts bleibt deshalb praktisch immer kleiner als die doppelte Materialstärke 16. Gegebenenfalls mit Ausnahme der Biegestellen.

Bei der Erstellung der 3D-Kontur und der Abwicklung, wie sie in Figur 5 dargestellt ist, wird darauf Rücksicht genommen, dass eine geeignete Biegeabfolge ermöglicht wird.

Insgesamt ergibt sich eine Rühreinrichtung bzw. ein Rührgerät 10 und ein damit ausgerüsteter Fermenter, welches bzw. welcher einen erheblich reduzierten Energiebedarf aufweist, da die dreidimensionale Kontur der Rührflügel gut an die theoretisch optimale Kontur angepasst werden können. Schon bei einer Anzahl von 4 und besser 6 oder 8 Umbiegungen wird eine effektive und dennoch kostengünstig herstellbare Rühreinrichtung zur Verfügung gestellt. Dabei sind sogar die einzelnen Rührflügel 11 bis 13 der Rühreinrichtung separat austauschbar.

Obwohl jeder einzelne Flügelabschnitt 21ff. aus einem ebenen Metallstreifen mit der Materialstärke 16 besteht, wird insgesamt eine strömungsgünstige und effektive Rühreinrichtung zur Verfügung gestellt.

Wenn bei dem Herstellverfahren und den einzelnen Biegeschritten geeignete Schablonen und Halterungen eingesetzt werden, kann auch bei einer händischen Fertigung ein effektiver Prozess ermöglicht werden.

**Bezugszeichenliste:**

| | | | |
|---|---|---|---|
| 1 | Fermenter | 25 | Flügelabschnitt |
| 2 | Fermenterwandung | 26 | Flügelabschnitt |
| 3 | Fermenterinnenraum | 27 | Flügelabschnitt |
| 4 | Horizontale | 28 | Flügelabschnitt |
| 5 | Fermenterdach | 29 | Flügelabschnitt |
| 6 | Serviceöffnung | 30 | Abstand |
| 7 | Substrat | 31 | Neigungswinkel |
| 8 | Führungseinheit | 32 | Oberflächenstelle |
| 9 | Stützeinheit | 33 | Achse |
| 10 | Rührgerät, Rühreinrichtung | 34 | Verbindungsmittel |
| | | 35 | Antriebseinrichtung |
| 11 | Rührflügel | 36 | Achsenaufnahme |
| 12 | Rührflügel | 37 | Trennlinie, Biegekante |
| 13 | Rührflügel | 37a | Winkel |
| 14 | Vielzahl | 38 | Ende |
| 15 | Grundkörper | 39 | Einstelleinheit |
| 16 | Materialstärke | 40 | Plattform |
| 17 | Höhe | 41 | Nabe, Flügelnabe |
| 18 | Flügelfläche | 43 | Flügelblatt |
| 19 | Drehachse | 44 | Flügelwurzel |
| 20 | Aufnahmeeinrichtung | 45 | axiale Erstreckung |
| 21 | Flügelabschnitt | 46 | radiale Erstreckung |
| 22 | Flügelabschnitt | 47 | radiale Länge |
| 23 | Flügelabschnitt | 100 | Biogasanlage |
| 24 | Flügelabschnitt | | |

## Patentansprüche

1. Rühreinrichtung (10) für einen Fermenter (1) einer Biogasanlage (100) mit einer Mehrzahl von Rührflügeln (11-13), wobei ein Rührflügel (11-13) jeweils eine Vielzahl (14) von zueinander gewinkelten Flügelabschnitten (21-29) umfasst,
**dadurch gekennzeichnet,**
**dass** die Flügelabschnitte (21-29) des Rührflügels (11-13) an den Trennlinien (37) zwischen den Flügelabschnitten (21-29) umgebogen und so zueinander gewinkelt sind, dass die Steigung des Rührflügels (11-13) mit steigendem Abstand (30) von einer zentralen Drehachse (19) abnimmt, um den Rührflügel (11-13) dreidimensional strömungsgünstig auszubilden, sodass der Vortrieb über der gesamten Querschnittsfläche sich nur wenig ändert, wobei die Biegekanten (37) in ihrer Ausrichtung neben einer Radialkomponente quer zu einer Drehachse (19) auch eine Axialkomponente parallel zu der Drehachse (19) aufweisen, wobei mit steigendem radialen Abstand einer Biegekante (37) von der Drehachse (19) die Axialkomponente der Biegekante (37) kleiner wird, wobei die Winkelbereiche zwischen zwei benachbarten Biegekanten (37) zwischen 0,5° und 3° liegen und wobei wenigstens fünf Flügelabschnitte (21-29) an dem Rührflügel (11-13) vorgesehen sind, wobei sich die Trennlinien (37) auf der Flügelfläche des Rührflügels nicht schneiden.

2. Rühreinrichtung (10) nach Anspruch 1, wobei die Flügelabschnitte (21-29) einstückig miteinander verbunden sind und wobei die Flügelabschnitte (21-29) durch vielfaches Umbiegen eines flachen Grundkörpers (15) gebildet sind, wobei der Grundkörper (15) aus einem metallischen Blechmaterial besteht und/oder wobei wenigstens ein Flügelabschnitt (21-29) abgekantet ist.

3. Rühreinrichtung (10) nach Anspruch 1 oder 2, wobei zwei benachbarte Flügelabschnitte jeweils eine dazwischen angeordnete und im Wesentlichen linear verlaufende Biegekante definieren.

4. Rühreinrichtung nach einem der vorhergehenden Ansprüche, wobei wenigstens ein Winkel (37a) zwischen zwei benachbarten Biegekanten (37) größer als 1° beträgt.

5. Rühreinrichtung nach einem der vorhergehenden Ansprüche, wobei die Rührflügel (11-13) an einer um eine Drehachse (19) drehbaren Flügelnabe (41) befestigt sind, wobei jeder Rührflügel (11-13) ein Flügelblatt (43) und eine Flügelwurzel (44) benachbart zu der Flügelnabe (41)umfasst.

6. Rühreinrichtung nach dem vorhergehenden Anspruch, wobei sich eine radial innere Flügelfläche (45) unter einem Winkel (46) zwischen 15° und 75° zu der Drehachse (19) der Flügelnabe (41) erstreckt.

7. Rühreinrichtung (10) nach einem der beiden vorhergehenden Ansprüche, wobei sich ein an der Flügelnabe (41) montierter Rührflügel (11-13) in axialer Richtung der Drehachse (19) über wenigstens 1/6 einer maximalen radialen Erstreckung (46) des Rührflügels von der Drehachse (19) aus erstreckt und/oder wobei wenigstens einer der Flügelabschnitte (21-29) eine Höhe (17) quer zu seiner Flügelfläche (18) aufweist, die kleiner als eine vierfache Materialstärke (16) des Flügelabschnitts (21-29) ist.

8. Rühreinrichtung (10) nach einem der vorhergehenden Ansprüche, wobei ein Produkt eines Abstandes (30) von einer zentralen Drehachse (19) und der lokalen Steigung des Rührflügels (11-13) sich über der Flügelfläche (18) um weniger als den Wert 4 und insbesondere um weniger als den Wert 2 ändert und/oder wobei ein lokaler Neigungswinkel (31) an einer Oberflächenstelle (32) des Rührflügels (11-13) relativ zu einer Achse (33), die durch die Oberflächenstelle (32) verläuft und quer zu der zentralen Drehachse (19) ausgerichtet ist, mit zunehmendem Abstand (30) von der zentralen Drehachse (19) abnimmt.

9. Rühreinrichtung (10) nach einem der vorhergehenden Ansprüche, wobei der Rührflügel (11-13) an einer Aufnahmeeinrichtung (20) befestigt ist und/oder wobei ein Mehrzahl von zwei, drei oder mehr Rührflügeln (11-13) vorgesehen ist.

10. Rühreinrichtung (10) nach Anspruch 9, wobei die Aufnahmeeinrichtungen (20) der Rührflügel (11-13) insgesamt eine mehrkantige Achsenaufnahme (36) bilden und/oder wobei der Rührflügel (11-13) über wenigstens ein lösbares Verbindungsmittel (34) an der Aufnahmeeinrichtung (20) befestigt ist.

11. Rühreinrichtung (10) nach einem der vorhergehenden Ansprüche, mit einer Antriebseinrichtung (35) und wenigstens einer Antriebswelle, wobei der Rührflügel (11-13) mit der Antriebswelle wenigstens im Wesentlichen drehfest verbunden ist.

12. Fermenter (1) einer Biogasanlage mit einer Fermenterwandung (2) und wenigstens einem Fermenterinnenraum (3) und wenigstens einem Fermenterdach (5) und mit wenigstens einer in dem Fermenterinnenraum (3) angeordneten Rühreinrichtung (10) nach einem der vorhergehenden Ansprüche.

13. Verfahren zur Herstellung einer Rühreinrichtung (10) nach einem der Ansprüche 1 bis 10 für einen Fermenter (1) einer Biogasanlage (10) mit einer Mehrzahl von Rührflügeln (11-13), wobei ein Rührflügel (11-13) jeweils eine Vielzahl (14) von zueinander gewinkelten Flügelabschnitten (21-29) umfasst
**dadurch gekennzeichnet,**
**dass** die Flügelabschnitte (21-29) der Rührflügel (11-13) an den Trennlinien (37) zwischen den Flügelabschnitten (21-29) so zueinander gewinkelt umgebogen werden, dass die Steigung des Rührflügels (11-13) mit steigendem Abstand (30) von einer zentralen Drehachse (19) abnimmt, und dass die Biegekanten (37) in ihrer Ausrichtung neben einer Radialkomponente quer zu einer Drehachse (19) auch eine Axialkomponente parallel zu der Drehachse (19) aufweisen, wobei mit steigendem radialen Abstand einer Biegekante (37) von der Drehachse (19) die Axialkomponente der Biegekante (37) kleiner wird und dass wenigstens ein Winkel (37a) zwischen zwei benachbarten Biegekanten (37) größer als 1° beträgt, um die Rührflügel (11-13) dreidimensional strömungsgünstig auszubilden.

14. Verfahren nach dem vorhergehenden Anspruch, wobei der Rührflügel (11-13) aus einem flächigen Grundkörper (15) durch Umbiegen an den einzelnen Flügelabschnitten (21-29) erzeugt wird und/oder wobei der flächige Grundkörper (15) an einer Trennlinie (37) zwischen zwei Flügelabschnitten (21-29) umgebogen und insbesondere abgekantet wird.

15. Verfahren nach einem der beiden vorhergehenden Ansprüche, wobei mehrere Rührflügel (11-13) über lösbare Verbindungsmittel (34) mittelbar oder unmittelbar miteinander verbunden werden und/oder wobei ein Winkel (37a) zwischen zwei Flügelabschnitten (21-29) von einem Abstand (30) von einer Drehachse (19) abhängt.

## Claims

1. Agitating device (10) for a digester (1) of a biogas plant (100) having a plurality of agitator blades (11-13), each agitator blade (11-13) comprising a plurality (14) of blade sections (21-29) angled relative to one another,
**characterized in**
**that** the blade sections (21-29) of the agitator blade (11-13) are bent over at an angle relative to one another on the separation lines (37) between the blade sections (21-29), such that the gradient of the agitator blade (11-13) decreases with the distance (30) from a central rotational axis (19) increasing so as to configure the agitator blade (11-13) in a flow-optimized, three-dimensional shape, so as to provide for little variations in the advancing drive over the entire cross-sectional area, wherein the bending edges (37) show in their orientation, other than a radial component transverse to a rotational axis (19), also an axial component in parallel to the rotational axis (19), wherein with the radial distance of a bending edge (37) from the rotational axis (19) increasing, the axial component of the bending edge (37) decreases, wherein preferred angles between pairs of adjacent bending edges (37) range between 0.5° and 3°, and wherein the agitator blade (11-13) is provided with at least five blade sections (21-29), wherein the separation lines (37) do not intersect on the blade surface 18 of the agitator blade.

2. The agitating device (10) according to claim 1, wherein the blade sections (21-29) are integrally connected with one another, and wherein the blade sections (21-29) are formed by bending over a flat basic body (15) multiple times, the basic body (15) consisting of a metallic sheet material, and/or wherein at least one blade section (21-29) is edge-bent.

3. The agitating device (10) according to claim 1 or 2, wherein pairs of adjacent blade sections define a bending edge disposed in-between and extending substantially linearly.

4. The agitating device (10) according to any of the preceding claims, wherein at least one angle (37a) between pairs of adjacent bending edges (37) is larger than 1°.

5. The agitating device according to any of the preceding claims, wherein the agitator blades (11-13) are attached to a blade hub (41) rotatable about a rotational axis (19), wherein each of the agitator blades (11-13) comprises a blade body (43) and a blade base (44) adjacent to the blade hub (41).

6. The agitating device according to the preceding claim, wherein a radially inwardly blade surface (45) extends at an angle (46) between 15° and 75° to the rotational axis (19) of the blade hub (41) .

7. The agitating device (10) according to any of the two preceding claims, wherein an agitator blade (11-13) mounted to the blade hub (41) extends in the axial direction of the rotational axis (19) over at least 1/6 of the maximum radial extension (46) of the agitator blade from the rotational axis (19), and/or wherein at least one of the blade sections (21-29) shows an elevation (17) transverse to its blade surface (18) that is smaller than four times the material thickness (16) of the blade section (21-29) .

8. The agitating device (10) according to any of the preceding claims, wherein a product of a distance (30) from a central rotational axis (19) and the local gradient of the agitator blade (11-13) varies over the blade surface (18) by less than the value of 4 and in particular by less than the value of 2, and/or wherein a local inclination angle (31) in a surface location (32) of the agitator blade (11-13) relative to an axis (33) extending through the surface location (32) and oriented transverse to the central rotational axis (19), decreases with the distance (30) from the central rotational axis (19) increasing.

9. The agitating device (10) according to any of the preceding claims, wherein the agitator blade (11-13) is attached to a mounting device (20), and/or wherein a plurality of two, three or more agitator blades (11-13) is provided.

10. The agitating device (10) according to claim 9, wherein the mounting devices (20) of the agitator blades (11-13) overall form a multi-edged axle mount (36), and/or wherein the agitator blade (11-13) is attached to the mounting device (20) by means of at least one detachable connecting device (34).

11. The agitating device (10) according to any of the preceding claims, having a driving device (35) and at least one drive shaft, wherein the agitator blade (11-13) is at least substantially non-rotatably connected with the drive shaft.

12. Digester (1) of a biogas plant having a digester wall (2) and at least one digester interior (3) and at least one digester roof (5) and at least one agitating device (10) disposed in the digester interior (3) according to any of the preceding claims.

13. Method for manufacturing an agitating device (10) according to any of the claims 1 to 10, for a digester (1) of a biogas plant (10) having a plurality of agitator blades (11-13), wherein each of the agitator blades (11-13) comprises a plurality (14) of blade sections (21-29) angled relative to one another,
**characterized in**
**that** the blade sections (21-29) of the agitator blades (11-13) are bent over at an angle relative to one another on the separation lines (37) between the blade sections (21-29), such that the gradient of the agitator blade (11-13) decreases with the distance (30) from a central rotational axis (19) increasing, and that the bending edges (37) show in their orientation, other than a radial component transverse to a rotational axis (19), also an axial component parallel to the rotational axis (19), wherein with the radial distance of a bending edge (37) from the rotational axis (19) increasing, the axial component of the bending edge (37) decreases, and that at least one angle (37a) between pairs of adjacent bending edges (37) is larger than 1°, so as to configure the agitator blades (11-13) in a flow-optimized, three-dimensional shape.

14. The method according to the preceding claim, wherein the agitator blade (11-13) is manufactured from a two-dimensional basic body (15) by bending over each of the blade sections (21-29), and/or wherein the two-dimensional basic body (15) is bent over and in particular edge-bent on a separation line (37) between two blade sections (21-29).

15. The method according to any of the two preceding claims, wherein multiple agitator blades (11-13) are interconnected indirectly or directly via detachable connecting devices (34), and/or wherein an angle (37a) between pairs of blade sections (21-29) depends on the distance (30) from a rotational axis (19).

## Revendications

1. Dispositif agitateur (10) pour un digesteur (1) d'une installation de biogaz (100) présentant une pluralité de pales d'agitateur (11-13), chaque pale d'agitateur (11-13) comprenant une pluralité (14) de parties de pale (21-29) disposées angulairement l'une par rapport à l'autre, **caractérisé en ce que** les parties de pale (21-29) de la pale d'agitateur (11-13) sont disposées angulairement les unes par rapport aux autres sur les arrêtes de séparation (37) entre les parties de pale (21-29) de telle sorte que la pente de la pale d'agitateur (11-13) diminue à mesure que la distance (30) à un axe central de rotation (19) augmente afin de configurer la pale d'agitateur (11-13) sous une forme tridimensionnelle optimisée en flux, de sorte que la propulsion ne change que peu sur toute la surface de la section, dans lequel les arrêtes de séparation (37) montrent dans leur orientation autre qu'une composante radiale traverse à un axe de rotation (19), aussi une composante axiale parallèle à l'axe de rotation (19), dans lequel à mesure que la distance radiale d'une arrête de séparation (37) à l'axe de rotation (19) augmente, la composante axiale de l'arrête de séparation (37) diminue, dans lequel l'angle entre deux arêtes de séparation (37) adjacentes se trouve entre 0,5° et 3° et dans lequel la pale d'agitateur (11-13) est pourvue d'au moins cinq parties de pale (21-29), dans lequel les lignes de séparation (37) ne se coupent pas sur la surface de pale de la pale d'agitateur.

2. Dispositif agitateur (10) selon la revendication 1, dans lequel les parties de pale (21-29) sont intégralement raccordées les unes aux autres et dans lequel les parties de pale (21-29) sont formées par de multiples courbures d'un corps de base non plat (15), le corps de base (15) étant constitué d'un matériau en feuille métallique, et/ou dans lequel au moins une partie de pale (21-29) est pliée au bord.

3. Dispositif agitateur (10) selon la revendication 1 ou 2, dans lequel des paires de parties de pale adjacentes définissent un bord courbé disposé entre elles et s'étendant essentiellement linéairement.

4. Dispositif agitateur selon l'une quelconque des revendications précédentes, dans lequel au moins un angle (37a) entre des paires d'arrêtes de séparation adjacentes (37) est supérieur à 1°.

5. Dispositif agitateur selon l'une quelconque des revendications précédentes, dans lequel les pales d'agitateur (11-13) sont fixées sur un moyeu de pale (41) rotatif autour d'un axe de rotation (19) dans lequel chacune des pales d'agitateur (11-13) comprend un corps de pale (43) et une base de pale (44) adjacents au moyeu de pales (41).

6. Dispositif agitateur selon l'une quelconque des revendications précédentes, dans lequel une surface radialement vers l'intérieur de la pale (45) s'étend avec un angle (46) entre 15° et 75° avec l'axe de rotation (19) du moyeu de pale (41).

7. Dispositif agitateur (10) selon l'une quelconque des revendications précédentes, dans lequel une pale d'agitateur (11-13) montée sur le moyeu de pale (41) s'étend dans la direction axiale de l'axe de rotation (19) sur au moins 1/6 de l'extension radiale maximum (46) de la pale d'agitateur à partir de l'axe de rotation (19), et/ou dans lequel au moins une des parties de pale (21-29) montre une élévation (17) transverse à sa surface de pale (18) qui est plus petite que quatre fois l'épaisseur du matériau (16) de la partie de pale (21-29).

8. Dispositif agitateur (10) selon l'une quelconque des revendications précédentes, dans lequel un produit d'une distance (30) à partir d'un axe central de rotation (19) et la pente locale de la pale d'agitateur (11-13) varie avec la surface de pale (18) de moins de la valeur de 4 et en particulier de moins de la valeur de 2, et/ou dans lequel un angle d'inclinaison local (31) à un point de surface (32) de la pale d'agitateur (11-13) par rapport à un axe (33) s'étendant à travers le point de surface (32) et orientée de manière transverse à l'axe central de rotation (19) diminue à mesure que la distance (30) à l'axe central de rotation (19) augmente.

9. Dispositif agitateur (10) selon l'une quelconque des revendications précédentes, dans lequel la pale d'agitateur (11-13) est fixée à un dispositif de réception (20), et/ou dans lequel une pluralité de deux, trois or plus de pales d'agitateur (11-13) sont pourvues.

10. Dispositif agitateur (10) selon la revendication 9, dans lequel le dispositif de réception (20) des pales d'agitateur (11-13) forme un montage à logement d'arbre polygonal (36), et/ou dans lequel la pale d'agitateur (11-13) est fixée au dispositif de réception (20) aux moyens d'au moins un dispositif de raccordement libérable (34).

11. Dispositif agitateur (10) selon l'une quelconque des revendications précédentes, présentant un dispositif d'entraînement (35) et au moins un arbre d'entraînement dans lequel la pale d'agitateur (11-13) est au moins sensiblement liée en rotation de manière fixe avec l'arbre d'entraînement.

12. Digesteur (1) d'une installation de biogaz présentant une paroi de digesteur (2) et au moins un espace intérieur du digesteur (3) et au moins un toit de digesteur (5) et au moins un dispositif agitateur (10) disposé dans l'espace l'intérieur du digesteur (3) selon l'une quelconque des revendications précédentes.

13. Procédé de fabrication d'un dispositif agitateur (10) selon l'une quelconque des revendications 1 à 10, pour un digesteur (1) d'une installation de biogaz (10) présentant une pluralité de pales d'agitateur (11-13), chacune des pales d'agitateur (11-13) comprenant une pluralité (14) de parties de pale (21-29) disposées angulairement les unes par rapport aux autres, **caractérisé en ce que** les parties de pale (21-29) des pales d'agitateur (11-13) sont disposées angulairement les unes par rapport aux autres sur les arrêtes de séparation (37) entre les parties de pale (21-29) de telle sorte que la pente de la pale d'agitateur (11-13) diminue à mesure que la distance (30) à un axe de central rotation (19) augmente, dans lequel les arrêtes de séparation (37) montrent dans leur orientation autre qu'une composante radiale traverse à un axe de rotation (19), aussi une composante axiale parallèle à l'axe de rotation (19), dans lequel à mesure que la distance radiale d'une arrête de séparation (37) à l'axe de rotation (19) augmente, la composante axiale de l'arrête de séparation (37) diminue et dans lequel au moins un angle (37a) entre deux arêtes de séparation adjacentes (37) est supérieur à 1°, afin de configurer les pales d'agitateur (11-13) sous une forme tridimensionnelle optimisée en flux.

14. Procédé selon la revendication précédente, dans lequel la pale d'agitateur (11-13) est fabriquée à partir d'un corps de base bidimensionnel (15) par courbure de chacune des parties de pale (21-29) et/ou dans lequel le corps de base bidimensionnel (15) est courbé et en particulier recourbé au niveau des bords sur une arrête de séparation (37) entre deux parties de pale (21-29).

15. Procédé selon l'une quelconque des deux revendications précédentes, dans lequel de multiples pales d'agitateur (11-13) sont raccordées entre elles indirectement ou directement via des dispositifs de raccordement libérables (34) et/ou dans lequel un angle (37a) entre des paires de parties de pale (21-29) dépend de la distance (30) à un axe de rotation (19).
